# EUROPEAN PATENT SPECIFICATION

(11) **EP 1 714 656 B1**
(45) Date of publication and mention of the grant of the patent: **16.08.2017**
(21) Application number: 05709801.4
(22) Date of filing: 07.02.2005
(51) Int. Cl.: A61K 31/593, A61K 47/44, A61K 47/22, A61K 9/48, A61P 19/10

(54) **ED-71 PREPARATION**
ED-71-PRÄPARAT
PREPARATION DE L'ED-71

(30) Priority: 06.02.2004 JP 2004030702
(43) Date of publication of application: 25.10.2006
(73) Proprietor: CHUGAI SEIYAKU KABUSHIKI KAISHA, Tokyo, 115-8543 (JP)
(72) Inventor: KATSUKI, Hisakazu, chome, Kita-ku, Tokyo 1158543 (JP); SHIBATA, Masaki c/o Chugai Seiyaku Kabushiki, Shizuoka 4128513 (JP); MURAMATSU, Kazunori, chome, Kita-ku, Tokyo 11585 43 (JP); MIZUTANI, Akihiko, 5-chome, Kita-ku, Tokyo 1158543 (JP); YAMAUCHI, Tsuyoshi c/o Chugai Seiyaku Kabushiki, Shizuoka 4260041 (JP); SUZUKI, Kouji c/o Chugai Seiyaku Kabushiki, Tokyo 1158543 (JP)
(74) Representative: Vossius & Partner Patentanwälte Rechtsanwälte mbB
(86) International application number: PCT/JP2005/001749
(87) International publication number: WO 2005/074943

(56) References cited:
- WO-A1-01/90061
- WO-A1-03/047595
- JP-A- 2 215 765
- JP-A- 5 004 925
- JP-A- 6 041 060
- JP-A- 6 087 750
- JP-A- 10 072 432
- JP-A- 63 107 929
- JP-A- 2002 505 668
- US-A1- 2003 170 324
- US-B1- 6 448 421
- DATABASE WPI Week 197811 Thomson Scientific, London, GB; AN 1978-20658A XP002684023, & JP 53 012414 A (CHUGAI PHARM CO LTD) 3 February 1978 (1978-02-03)

## Description

### TECHNICAL FIELD

The present invention relates to a preparation comprising (5Z,7E)-(1R,2R,3R)-2-(3-hydroxypropoxy)-9,10-secocholesta-5,7,10(19)-triene-1,3,25-triol (hereinafter, also referred to as "ED-71"). The present invention also relates to a trans isomer of ED-71, (5E,7E)-(1R,2R,3R)-2-(3-hydroxypropoxy)-9,10-secocholesta-5,7,10(19)-triene-1,3,25-triol (hereinafter, also referred to as a "trans form").

### BACKGROUND ART

(5Z,7E)-(1R,2R,3R)-2-(3-hydroxypropoxy)-9,10-secocholesta-5,7,10(19)-triene-1,3,25-triol is a synthetic derivative of active vitamin D₃ with bone formation action developed by Chugai Pharmaceutical Co., Ltd. and is a drug that is now undergoing clinical testing as a therapeutic drug for osteoporosis (Bone, Vol. 30 (4), 582-588, 2002).

To produce ED-71 preparations, approaches for producing preparations such as soft capsules may be utilized, as in vitamin D derivatives known in the art. For example, it has been reported relative to vitamin D derivative preparations known in the art that 1α,25-dihydroxycholecalciferol can be maintained stably by adding 0.01 to 5% by weight of tocopherols to a pharmaceutical composition comprising the 1α,25-dihydroxycholecalciferol (Japanese Patent Laid-Open No. 6-87750) and that the storage stability of alfacalcidol can be enhanced by adding dl-α-tocopherol and dibutylhydroxytoluene at a 1:1 weight ratio and in an amount of 0.005% or more in total as antioxidants to a soft capsule comprising the alfacalcidol (Japanese Patent Laid-Open No. 5-4925). However, neither of the disclosures mentions whether the generation of degradation products of vitamin D groups can be suppressed.

According to the guideline published by Ministry of Health, Labour and Welfare Japan (ICH Harmonized Tripartite Guideline (Impurities in New Drug Products, Q3B(R), ICH Steering Committee, 2003), the confirmation of safety of a degradation product is strictly required when the content of the degradation product in a preparation exceeds 1%. Thus, it is important for the production of drug preparations that the content of each of the degradation products in the preparation does not exceed 1%.

Therefore, to produce ED-71 preparations, it is also important in practice to not only enhance the storage stability of ED-71 serving as an active ingredient but also to suppress the generation of main degradation products.

WO 03/047595 discloses the use of antioxidants for suppressing the oxidation of an active vitamin D compound.

US 6,448,421 discloses purified crystals of the vitamin D derivative of ED-71.

In US 2003/0170324 A1, an oily liquid preparation with increased stability is described, comprising an antioxidant and a 25-hydroxy vitamin D3, described as being prone to oxidation.

JP 53-12414 A discloses that an oily solution of 1α-hydroxy vitamins may be stabilised by adding a small amount of one or more of BHT, a gallate, and a tocopherol.

### DISCLOSURE OF THE INVENTION

### PROBLEMS TO BE SOLVED BY THE INVENTION

An object of the present invention is to provide a method of suppressing isomerization as defined in independent claim 1 occurring in an oily preparation comprising ED-71.

### MEANS FOR SOLVING THE PROBLEMS

The present inventors have conducted extensive studies for solving the problems and have consequently found that main degradation products of ED-71 in fat and oil are a tachysterol-type isomer of ED-71 represented by the following structural formula:

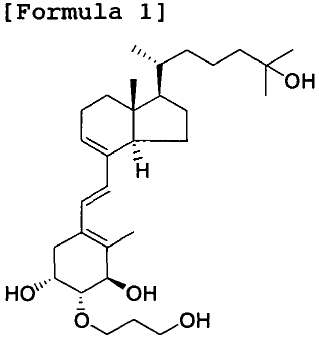

(6E-(1R,2R,3R)-2-(3-hydroxypropoxy)-9,10-secocholesta-5(10),6,8(9)-triene-1,3,25-triol; hereinafter, also referred to as "tachysterol form" of ED-71), and a trans form of ED-71 represented by the following structural formula:

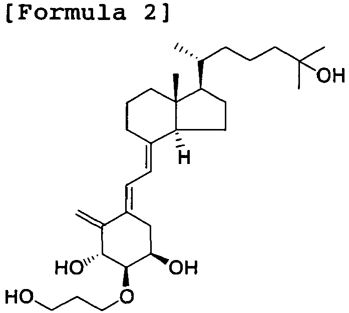

((5E,7E)-(1R,2R,3R)-2-(3-hydroxypropoxy)-9,10-secocholesta-5,7,10(19)-triene-1,3,25-triol). The present inventors have completed the present invention by further finding that the generation of these degradation products can be suppressed in an oily preparation comprising ED-71 by adding an antioxidant as defined in independent claim 1 thereto.

In the preparation, preferably, the generation of a degradation product of (5Z,7E)-(1R,2R,3R)-2-(3-hydroxypropoxy)-9,10-secocholesta-5,7,10(19)-triene-1,3,25-triol is suppressed. The degradation product of (5Z,7E)-(1R,2R,3R)-2-(3-hydroxypropoxy)-9,10-secocholesta-5,7,10(19)-triene-1,3,25-triol may be 6E-(1R,2R,3R)-2-(3-hydroxypropoxy)-9,10-secocholesta-5(10),6,8(9)-triene-1,3,25-triol and/or (5E,7E)-(1R,2R,3R)-2-(3-hydroxypropoxy)-9,10-secocholesta-5,7,10(19)-triene-1,3,25-triol. Preferably, the degradation product of (5Z,7E)-(1R,2R,3R)-2-(3-hydroxypropoxy)-9,10-secocholesta-5,7,10(19)-triene-1,3,25-triol is 6E-(1R,2R,3R)-2-(3-hydroxypropoxy)-9,10-secocholesta-5(10),6,8(9)-triene-1,3,25-triol and (5E,7E)-(1R,2R,3R)-2-(3-hydroxypropoxy)-9,10-secocholesta-5,7,10(19)-triene-1,3,25-triol.

In the preparation, preferably, the degradation of (5Z,7E)-(1R,2R,3R)-2-(3-hydroxypropoxy)-9,10-secocholesta-5,7,10(19)-triene-1,3,25-triol into 6E-(1R,2R,3R)-2-(3-hydroxypropoxy)-9,10-secocholesta-5(10),6,8(9)-triene-1,3,25-triol and/or (5E,7E)-(1R,2R,3R)-2-(3-hydroxypropoxy)-9,10-secocholesta-5,7,10(19)-triene-1,3,25-triol is suppressed.

In the preparation, preferably, the antioxidant is one member selected from butylhydroxyanisole, catechin, tocopherol, dibutylhydroxytoluene, and citric acid. More preferably, the antioxidant is the dl-α-tocopherol.

Preferably, the preparation is an oily preparation. More preferably, the preparation is a soft capsule, hard capsule, or oily liquid preparation (particularly, a soft capsule).

The preparation should comprise 0.000001 to 0.01% by weight of (5Z,7E)-(1R,2R,3R)-2-(3-hydroxypropoxy)-9,10-secocholesta-5,7,10(19)-triene-1,3,25-triol relative to the fat and oil and 0.0001 to 12% by weight of the antioxidant relative to the fat and oil.

### ADVANTAGE OF THE INVENTION

The method of the present invention makes it possible to provide a pharmaceutical formulation capable of suppressing the generation of a degradation product of ED-71.

### BRIEF DESCRIPTION OF THE DRAWINGS

Figure 1 shows a UV-HPLC chromatograph (265 nm) of a drug solution that has been left for 33 days under the condition of 40°C/75% RH; and
Figure 2 shows an RI-HPLC chromatograph of the drug solution that has been left for 33 days under the condition of 40°C/75% RH.

### BEST MODE FOR CARRYING OUT THE INVENTION

Hereinafter, a more specific aspect of the present invention and a method for practicing the present invention will be described.

(5Z,7E)-(1R,2R,3R)-2-(3-hydroxypropoxy)-9,10-secocholesta-5,7,10(19)-triene-1,3,25-triol (ED-71) is a compound represented by the following structural formula:

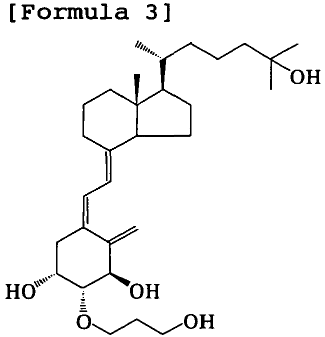

The ED-71 can be obtained, for example, in accordance with the method described in Japanese Patent Laid-Open No. 10-72432, by subjecting (1R,2R,3R)-2-(3-hydroxypropoxy)cholesta-5,7-diene-1,3,25-triol used as a starting material to ultraviolet irradiation and thermal isomerization reaction and then to purification by reverse phase HPLC and concentration, followed by crystallization with ethyl acetate.

An "antioxidant" used in the present invention includes butylhydroxyanisole (BHA), catechin, tocopherol, dibutylhydroxytoluene (BHT), and citric acid. These antioxidants can be used alone or in combination of 2 or more of them. The antioxidant is preferably dl-α-tocopherol.

"Fat and oil" used in the present invention include medium-chain triglyceride (hereinafter, also referred to as "MCT"), tricaprilin, caproic acid, caprylic acid, capric acid, oleic acid, linoleic acid, linolenic acid, and plant oil. In this context, the plant oil includes coconut oil, olive oil, rapeseed oil, peanut oil, corn oil, soybean oil, cottonseed oil, grape oil, and safflower oil. The fat and oil are preferably MCT, tricaprilin, caproic acid, caprylic acid, or capric acid free of unsaturated fatty acid, particularly preferably MCT.

In the present invention, "degradation product of ED-71" refers to main degradation products detected during the storage of ED-71 as an oily preparation and is specifically the tachysterol and the trans forms of ED-71.

The tachysterol form of ED-71 is a compound which has tachysterol skeleton and which is represented by the following structural formula, and whose chemical name is 6E-(1R,2R,3R)-2-(3-hydroxypropoxy)-9,10-secocholesta-5(10),6,8(9)-triene-1,3,25-triol:

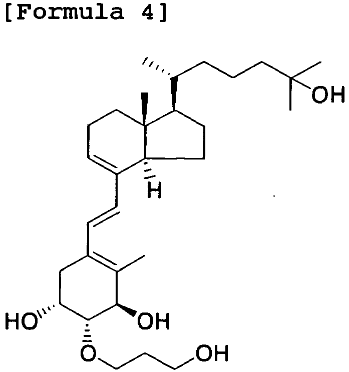

The tachysterol form can be synthesized in accordance with the method described in, for example Japanese Patent Laid-Open No. 10-72432, and can also be synthesized by the following method: the tachysterol form can be obtained by subjecting a tetrahydrofuran solution of (1R,2R,3R)-2-(3-hydroxypropoxy)-cholesta-5,7-diene-1,3,25-triol used as a starting material to ultraviolet irradiation at a low temperature under an argon atmosphere, and followed by purification by reverse phase HPLC and concentration to dryness.

The trans form of ED-71 is a compound which has a trans double bond at the 5-6 position, which is represented by the following structural formula, and whose chemical name is (5E,7E)-(1R,2R,3R)-2-(3-hydroxypropoxy)-9,10-secocholesta-5,7,10(19)-triene-1,3,25-triol:

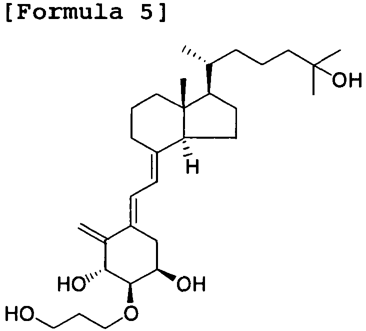

As is described above, it is important for the production of drug preparations that the content of each degradation product of an active ingredient does not exceed 1%.

According to the studies conducted by the present inventors, after an ED-71 preparation wherein a solution of ED-71 dissolved in MCT is encapsulated in a soft capsule was stored for 12 months at 30°C, that is the upper limit of "room temperature", the amounts of both the tachysterol and trans forms generated exceeded 1%(see Tables 2 and 3 described below). Therefore, in producing ED-71 preparations, it is required to suppress the generation of these major degradation products. Namely, to put the ED-71 preparation into practical use, it is important to not only prevent the degradation of the ED-71 but also, preferably, to prevent the degradation thereof into the tachysterol and trans forms.

The existence of the tachysterol and trans forms can be confirmed by detecting them by reverse phase liquid chromatography and measuring the absorbance at a measurement wavelength of 265 nm.

In a state in which the generation of the degradation product of ED-71 is suppressed, the amounts of each of the tachysterol and trans forms generated after 12-month storage at room temperature under shading is preferably 1% or less, more preferably 0.3% or less, and particularly preferably 0.1% or less.

In the present invention, "oily preparation" refers to an orally administrable preparation produced from a drug solution in fat and oil.

The oily preparation available in the present invention includes a soft capsule, hard capsule, and oily liquid preparation.

A soft capsule refers to a preparation where a mixture solution of a drug dissolved in fat or oil is enclosed in a shell mainly composed of a film-forming component such as gelatin. Any method for production of a soft capsule, such as a dripping method or a rotary-die method, can be used in the present invention, as long as the method is capable of producing soft capsules. The dripping method utilizes such a principle that a two-phase fluid flow of a substance serving as a capsule core and a substance serving as a capsule shell, when these are simultaneously dripped from a nozzle into a coolant, is changed into droplets by interfacial tension, which are cooled to foam capsules having hardened shells. In the rotary-die method, sheets (sheets containing a gel-forming component such as gelatin) continuously pass through two counter-rotating formation rollers, which in turn stamp capsule-forming bodies out of the sheets while filling material is injected into the space between two stamped-out sections which are then welded at their ends by thermal action to form a seamed soft capsule. Soft capsules of vitamin D's and their production methods include those described in International Publication Nos. WO01/15702, WO02/13755, and WO03/094897, and International Patent Application No. PCT/JP03/07885.

A hard capsule is a preparation wherein a solution containing a drug is filled into a shell capsule consisting of a cap and a body which are mainly composed of a film-forming component such as gelatin, and wherein an overlapping portion of the cap and the body is sealed by applying a gelatin solution or the like thereto, to prevent the leakage of the solution.

The shells of the soft capsules and the hard capsules are composed of a film-forming component, a plasticizer, a shading agent, and so on, which are mixed together.

A film-forming component may be an animal-derived component such as various types of gelatins or a non-animal-derived component such as various types of watersoluble polymers. One or more of these components can be mixed at an arbitrary ratio for use in a film. An animal-derived component includes alkali-treated gelatin, acid-treated gelatin, and chemically modified gelatin. The chemically modified gelatin that can be used in the present invention is not particularly limited by its modification manner and may be those produced by reacting an amino group of gelatin with a substance such as succinic acid, phthalic acid, and acetic acid. The gelatin used for the chemically modified gelatin may also be alkali-treated gelatin or acid-treated gelatin. The non-animal-derived component includes: polysaccharides extracted from seaweed such as agar, carrageenan, and alginic acid; polysaccharides obtained from plant seeds such as locust bean gum, guar gum, tamarind gum, Cassia gum, and tara bean gum; polysaccharides secreted by plants such as gum arabic, tragacanth gum, almond gum, and damson gum; polysaccharides extracted from plants such as pectin, arabinogalactan, and glucomannan; polysaccharides obtained from microorganisms such as gellan gum, xanthan gum, pullulan, dextran, and curdlan; and cellulose gums such as crystalline cellulose, methylcellulose, carboxymethylcellulose and hydroxypropylmethylcellulose.

A plasticizer includes glycerin, sorbitol, maltose, glucose, maltitose, sucrose, xylitol, mannitol, erythritol, and polyethylene glycols (molecular weight: 400 to 6000). In the present invention, one or more of these plasticizers can be used.

A shading agent includes: metallic oxides such as titanium oxide, iron sesquioxide (colcothar), yellow ferric oxide, yellow iron oxide, and zinc oxide; inorganic compounds such as talc, calcium carbonate, magnesium carbonate, magnesium silicate, and light silicic anhydride; and food dyes such as caramel, food red No. 3 aluminum lake, food yellow No. 4 aluminum lake, food yellow No. 5 aluminum lake, food green No. 3 aluminum lake, food blue No. 2 aluminum lake, and sodium copper chlorophyllin. In the present invention, one or more of these water-insoluble shading agents can be used.

"Oily liquid preparation" refers to a preparation wherein an oily solution containing a drug is filled into a hermetically sealed container (e.g., a glass container, a plastic container, and a stick packaging container).

The amount of fat and oil used in the preparation of the present invention is not particularly limited. However, the amount is preferably 50 to 500 mg, particularly preferably 60 to 250 mg, per capsule (soft capsule and hard capsules). The amount of the fat and oil used in an oily liquid preparation is preferably 0.5 to 5 g, particularly preferably 1 to 3 g, per container.

The content of ED-71 contained in a preparation is further defined in independent claim 1. The amount of ED-71 per unit preparation is preferably 0.05 to 5 µg, particularly preferably 0.5 to 0.75 µg. When this amount is converted to an ED-71 concentration in fat and oil, the concentration for a capsule is preferably 0.00001% or more by weight, particularly preferably 0.0002% or more by weight to preferably 0.01% or less by weight, particularly preferably 0.00125% or less by weight. For an oily liquid preparation, this concentration is preferably 0.000001% or more by weight, particularly preferably 0.000017% or more by weight to preferably 0.001% or less by weight, particularly preferably 0.000075% or less by weight.

The amount of an antioxidant that can be mixed into fat or oil is further defined in independent claim 1. In general, an antioxidant can be used in amounts not higher than the maximum usage amount that is tolerated by antioxidants (e.g., amounts not higher than the maximum usage amount already approved and described in Pharmaceutical Additive Dictionary (Yakuji Nippo, 2000) or not higher than the limit of usage described in The Japan's Specifications and Standards for Food Additives (Japan Food Additives Association, 1999)).

Among others, dl-α-tocopherol is contained in fat and oil in an amount of preferably 0.0001% or more by weight, more preferably 0.002% or more by weight, particularly preferably 0.01% or more by weight, to preferably 12% or less by weight, more preferably 1% or less by weight, particularly preferably 0.1% or less by weight, in capsules. In an oily liquid preparation, preferably 0.0001% or more by weight, more preferably 0.002% or more by weight, particularly preferably 0.01% or more by weight to preferably 1.2% or less by weight, more preferably 1% or less by weight, particularly preferably 0.1% or less by weight of dl-α-tocopherol is contained in fat and oil. Dibutylhydroxytoluene, butylhydroxyanisole, propyl gallate, and the like are the same as in the above case of dl-α-tocopherol.

### Examples

Hereinafter, the present invention will be described more fully with reference to Examples. However, the present invention is not intended to be limited to these Examples by any means. In Examples below, ED-71 and a tachysterol form used were synthesized by the method described in Japanese Patent Laid-Open No. 10-72432.

### Example 1: Synthesis of the trans form ((5E,7E)-(1R,2R,3R)-2-(3-hydroxypropoxy)-9,10-secocholesta-5,7,10(19)-triene-1,3,25-triol) and its physical property data

### (Synthesis and purification procedures)

ED-71 (500 mg) was placed into a 50-mL eggplant-shaped flask under a nitrogen atmosphere, to which 30 mL of liquid sulfur dioxide was injected and refluxed (approximately at -15°C) with stirring for 3 hours. The sulfur dioxide was distilled off to obtain 560 mg of (5Z,7E)-(1R,2R,3R)-2-(3-hydroxypropoxy)-6,19-sulfone-9,10-secocholesta-5(10),7-diene-1,3,25-triol (hereinafter, referred to as a "sulfur dioxide adduct") as a pale yellow solid. The sulfur dioxide adduct (200 mg), 500 mg of sodium hydrogencarbonate, and 20 mL of ethanol were added to a 50-mL eggplant-shaped flask and refluxed under stirring for 210 minutes. After cooling, insoluble matter was filtered out, and the filtrate was concentrated to dryness. The dried concentrate was dissolved in 10 mL of ethanol and 10 mL of diethylether and filtered, and then the resulting filtrate was concentrated to dryness.

The dried concentrate was developed and treated in preparative reverse phase HPLC (apparatus: manufactured by Hitachi, Ltd., column: Kromasil ODS (KR100-7-C18, 250 x 20 mm I.D., manufactured by Eka Chemicals), mobile phase: 50%-acetonitrile solution, flow rate: 9.99 mL/min, detection: UV=220 nm and 265 nm) to fractionate a portion eluted from 54 minutes to 68 minutes. The fractionated solution was concentrated to dryness to obtain approximately 50 mg of white solid. The obtained white solid was dissolved in a small amount of ethanol and 0.5 mL of ethyl acetate and crystallized in a freezing chamber set at -20°C. A crystal thus precipitated was separated, then washed with a trace amount of ethyl acetate, and dried to obtain 39 mg of the trans form (yield: 22%).

### (Physical property data)

HPLC purity: 99.6% (HPLC conditions: Kromasil ODS 100-5C18, 5 µm, 4.6 mm I.D. x 250 mm, 55% acetonitrile solution, flow rate: 0.9 mL/min, 220 nm, 1 mg/mL 10 µL, measured area range: 4 to 30 min).
¹H-NMR (CDCl₃) (ppm): 6.63 (1H, d, J = 11.2 Hz), 5.85 (1H, d, J = 11.6 Hz), 5.18 (1H, t, J = 1.8 Hz), 5.13 (1H, t, J = 1.8 Hz), 4.43 (1H, d, j = 8.3 Hz), 4.28 (1H, ddd, J = 3.6 Hz, 3.6 Hz, 3.6 Hz), 3.7-4.0 (3H, m), 3.34 (1H, dd, J = 2.8 Hz, 8.4 Hz), 1.21 (6H, s), 0.93 (3H, d, J = 6.3 Hz), 0.56 (3H, s).
¹³C-NMR (CDCl₃) (ppm): 149.2, 145.4, 132.0, 123.4, 116.0, 109.0, 84.2, 71.3, 71.1, 66.3, 61.2, 56.5, 45.9, 44.4, 40.5, 36.4, 36.0, 31.8, 29.3, 29.2, 29.1, 27.6, 23.5, 22.3, 20.8, 18.8, 18.4, 12.2.
UV (ethanol) (λmax): 209.8 nm (ε12300), 274.6 nm (ε 23200).
IR (KBr) (cm⁻¹): 3365, 2951, 2935, 2877, 2845, 1628, 1468, 1458, 1437, 1375, 1363, 1350, 1333, 1215, 1120, 1080, 1059, 1034, 997, 958, 947, 933, 904, 891, 872, 729, 715, 683, 638, 627.

### Example 2: Identification of main degradation products in soft capsule comprising ED-71

### (Procedures)

Tritium-labeled ED-71 (2β-([3-³H]-3-hydroxypropyl)-1a,3β-25-trihydroxycholesta-5,7,10(19)-triene (alternatively designated as (5Z,7E)-(1R,2R,3R)-2-([3-³H]-3-hydroxypropoxy)-9,10-secocholesta-5,7,10(19)-triene-1,3,25-triol, hereinafter referred to as "³H-ED-71")) was used (J. Labeled Cpd. Radiopharm. 42, 519-525 (1999)) and dissolved together with unlabeled ED-71 in MCT (ED-71 concentration: 1 µg/100 mg, ³H-ED-71 concentration: 1.38 MBq/mL). This drug solution was filled (0.08 mL/capsule) into empty soft capsules (which had a shell consisting of 47.67 mg of gelatin, 16.68 mg of glycerin, and 0.65 mg of titanium oxide and were produced by a rotary die method) by means of a syringe with a needle. The capsules were sealed with gelatin and left for 33 days under 40°C/75% RH and shading. Degradation products generated in the drug solution were detected with HPLC (manufactured by Shimadzu) equipped with UV and RI detectors.

### HPLC analysis conditions

Column: YMC-Pack ODS AM-303 (250 x 4.6 mm, 5 µm), manufactured by YMC Co., Ltd.
Mobile phase: acetonitrile/water =1:1
Flow rate: 1.2 mL/min
Peak detection: UV 265 nm, RI
Column temperature: 30°C

### (Results)

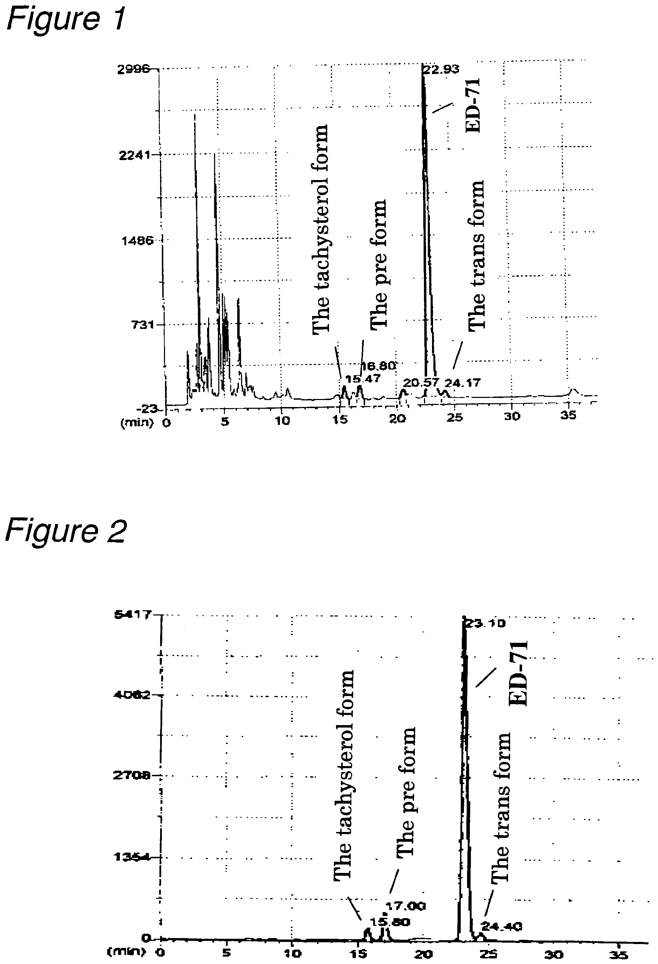

The chromatograms obtained by HPLC measurement of the drug solution that has been left for 33 days under the conditions of 40°C/75% RH are shown in Figures 1 and 2. In the RI-chromatogram, minor peaks were detected at around both 16 minutes and 24 minutes, in addition to a main peak corresponding to ED-71 and a minor peak corresponding to the pre form of ED-71 at around 17 minutes. These two peaks are not detected in a sample before storage under 40°C/75% RH, and the peaks detected in RI-HPLC are derived from ³H-ED-71. Therefore, it was suggested that these two components are main degradation products of ED-71. The peak eluted at around 16 minutes was almost identical to the position of the main eluted peak detected in the analysis of a standard solution of the tachysterol form under the same HPLC conditions, and is therefore considered to be derived from the tachysterol form. The peak eluted at around 24 minutes was almost identical to the position of the main eluted peak detected in the analysis of a standard solution of the trans form under the same HPLC conditions, and is therefore considered to be derived from the trans form.

The pre form of ED-71 (chemical name: 6Z-(1R,2R,3R)-2-(3-hydroxypropoxy)-9,10-secocholesta-5(10),6.8(9)-triene-1,3,25-triol) is a compound existing as an isomer having an equilibrium relationship with ED-71 in an oily or aqueous solution or in an organic solvent such as ethanol (Japanese Patent Laid-Open No. 10-72432) and is represented by the following structural formula:

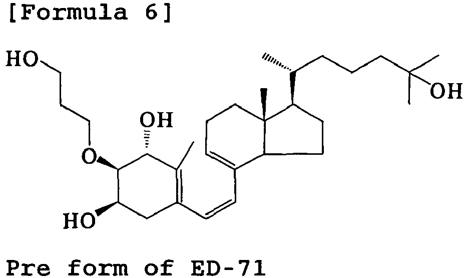

### Example 3:

To examine the influence of an antioxidant on the generation of the main degradation products in a preparation comprising ED-71, soft capsules filled with an MCT solution containing ED-71 and an antioxidant were stored under various types of conditions and evaluated for the generation behavior of the tachysterol and trans forms.

### (Formulation)

The formulation of the test soft capsule is shown in Table 1. ED-71 was mixed in the formulation in an amount of 0.0001% by weight relative to MCT. dl-α-tocopherol (manufactured by Wako Pure Chemical Industries, Ltd.) and BHT (dibutylhydroxytoluene) (manufactured by Wako Pure Chemical Industries, Ltd.) were used alone or in combination as an antioxidant. The amount of each of the antioxidants added was set to 0.02% by weight relative to MCT. Formulations containing the antioxidant(s) were defined as Formulations 1, 2, and 3, while a formulation free of the antioxidant was defined as Control Formulation 1.
[Table 1]

**Table 1 Formulation of soft capsule**

| Material name | Control Formulation 1 | Formulation 1 | Formulation 2 | Formulation 3 |
|---|---|---|---|---|
| | Antioxidant-free | BHT-added | Tocopherol-added | BHT+ tocopherol-added |
| Capsule shell | | | | |
| Gelatin (APB-H) | 47.67 mg | | | |
| Glycerin | 16.68 mg | | | |
| Titanium oxide | 0.65 mg | | | |
| Purified water | | | | |
| Subtotal | 65.00 mg | | | |
| Core solution | | | | |
| ED-71 | 1 µg | 1 µg | 1 µg | 1 µg |
| Anhydrous ethanol | 1.30 mg | 1.30 mg | 1.30 mg | 1.30 mg |
| BHT | | 0.02 mg | | 0.02 mg |
| dl-α-tocopherol | | | 0.02 mg | 0.02 mg |
| MCT(ODO-C) | 98.70 mg | 98.68 mg | 98.68 mg | 98.66 mg |
| Subtotal | 100.00 mg | 100.00 mg | 100.00 mg | 100.00 mg |
| Total | 165.00 mg | 165.00 mg | 165.00 mg | 165.00 mg |

### (Production procedures for soft capsule)

The soft capsules were produced by a dripping method by means of a seamless capsule filling machine (SPHEREX, manufactured by Freund Corporation) so that weights of the drug solution and the shell were 100 mg and 65 mg, respectively, per capsule.

### (Storage conditions)

Approximately 100 capsules each of the soft capsules were put in a vial. Then, the vials were hermetically sealed or unsealed and stored for 12 months under the conditions of 30°C/60% RH and shading.

### (Confirmation procedures for the tachysterol and trans forms)

After the termination of the storage period, the drug solution was extracted from each of the soft capsules, and a 50-µL aliquot thereof was subjected to HPLC analysis.

Column: YMC-Pack ODS AM-303 (250 x 4.6 mm, 5 µm), manufactured by YMC Co., Ltd.
Mobile phase: acetonitrile/water =1:1
Flow rate: 1.2 mL/min
Peak detection: 265 nm
Column temperature: 30°C

A peak area ratio of the tachysterol or trans form relative to the total sum of detected peak areas was calculated and used as an index of the amount of the tachysterol or trans form generated.

### (Measurement procedures for ED-71 content)

The content of ED-71 was measured by an HPLC method shown below to determine a residual rate after terminating the storage period.

### • Preparation procedures for internal standard solution:

Approximately 2 mg of heptyl 4-hydroxybenzoate was precisely weighed, and ethanol was added to volume 100 mL accurately. This solution was diluted 10-fold with ethanol and used as an internal standard solution.

### • Preparation procedures for standard solution:

Approximately 2 mg of a standard of ED-71 was precisely weighed, and ethanol was added to volume 200 mL accurately. A precisely 2.5 mL aliquot was taken from this solution, and ethanol was added to volume 20 mL accurately. The resulting solution was used as a standard undiluted solution.

Approximately 300 mg of MCT was placed into a 10-mL pear-shaped flask, to which 2 mL of the standard undiluted solution and 2 mL of the internal standard solution were then added. This mixture solution was dried under a reduced pressure in an evaporator at room temperature for 10 or more minutes. The remaining MCT solution was used as a standard solution.

### • Preparation procedures for sample solution:

The drug solutions were extracted from the capsules, and approximately 300 mg was precisely weighed and placed into a 10-mL pear-shaped flask. To these flask solutions, precisely 2 mL of the internal standard solution was added, and then dried under a reduced pressure in an evaporator at room temperature for 10 minutes. The remaining MCT solution was used as a sample solution.

### • HPLC analysis conditions:

The sample solutions and the standard solution (50 µL each) were subjected to column-switching HPLC analysis under the conditions described below.
Column: YMC-Pack ODS AM-303 (250 x 4.6 mm, 5 µm), manufactured by YMC Co., Ltd.
Mobile phase: (precolumn) MCT is eluted in a gradient using an acetonitrile/water (1:1) mixture solution as Solution A and acetonitrile as Solution B.
(Analysis column): acetonitrile/water (1:1) mixture solution
Flow rate: 1.2 mL/min
Detection wavelength: 265 nm
Column temperature: 23°C

### (Results)

The detection results of the tachysterol form, the trans form, and ED-71 by HPLC are shown in Tables 2 to 4.
[Table 2]

**Table 2 Peak area ratio of tachysterol form after storage of a hermetically sealed or unsealed vial storing a soft capsule containing 1 µg of ED-71 under 30°C/60% RH and shading**

| | | Peak area ratio of tachysterol form | | | |
|---|---|---|---|---|---|
| | | Control Formulation 1 | Formulation 1 | Formulation 2 | Formulation 3 |
| Storage conditions | Storage period (month) | Antioxidant -free | BHT | Tocopherol | BHT+ tocopherol |
| 30°C/60%RH Hermetically sealed | 0 | N.D. | N.D. | N.D. | N.D. |
| | 6 | 1.31 | N.D. | N.D. | N.D. |
| | 12 | 1.85 | 0.56 | N.D. | N.D. |
| 30°C/60%RH Unsealed | 0 | N.D. | N.D. | N.D. | N.D. |
| | 6 | 1.54 | N.D. | N.D. | N.D. |
| | 12 | 2.06 | 0.63 | N.D. | N.D. |

| | | | | | |
|---|---|---|---|---|---|
| n = 1, N.D. < 0.1%[0075] | | | | | |

[Table 3]

**Table 3 Peak area ratio of the trans form after storage of a hermetically sealed or unsealed vial storing a soft capsule containing 1 µg of ED-71 under 30°C/60% RH and shading**

| | | Peak area ratio of the trans form | | | |
|---|---|---|---|---|---|
| | | Control Formulation 1 | Formulation 1 | Formulation 2 | Formulation 3 |
| Storage conditions | Storage period (month) | Antioxidant -free | BHT | Tocopherol | BHT+ tocopherol |
| 30°C/60%RH Hermetically sealed | 0 | N.D. | N.D. | N.D. | N.D. |
| | 6 | 1.31 | N.D. | N.D. | N.D. |
| | 12 | 1.85 | 0.56 | N.D. | N.D. |
| 30°C/60%RH Unsealed | 0 | N.D. | N.D. | N.D. | N.D. |
| | 6 | 1.54 | N.D. | N.D. | N.D. |
| | 12 | 2.06 | 0.63 | N.D. | N.D. |

| | | | | | |
|---|---|---|---|---|---|
| n = 1, N.D. < 0.1% | | | | | |

[Table 4]

**Table 4 Residual rates of ED-71 after storage of a hermetically sealed or unsealed vial storing a soft capsule containing 1 µg of ED-71 under 30°C/60% RH and shading**

| | | Residual rate of ED-71 (%) | | | |
|---|---|---|---|---|---|
| | | Control Formulation 1 | Formulation 1 | Formulation 2 | Formulation 3 |
| Storage condition | Storage period (month) | Antioxidant -free | BHT | Tocopherol | BHT+ tocopherol |
| 30°C/60%RH Hermetically sealed | 6 | 96.7 | 99.9 | 100.5 | 100.0 |
| | 12 | 93.0 | 98.3 | 98.3 | 98.6 |
| 30°C/60%RH Unsealed | 6 | 93.6 | 98.0 | 97.6 | 97.9 |
| | 12 | 88.1 | 93.7 | 95.0 | 95.5 |

| | | | | | |
|---|---|---|---|---|---|
| n = 1 | | | | | |

As can be seen from Tables 2 to 3, the amounts of the tachysterol and trans forms generated in the preparation of the control formulation during 12-month storage in the unsealed state were 1.741% and 2.06%, respectively, in terms of the peak area ratio, whereas the amounts in both formulations 2 and 3 were suppressed to the detection limit (0.1%) or lower. As can be seen from Table 4, the residual rates of ED-71 in Formulations 1, 2, and 3 were higher than that in the control formulation. The results showed that the generation of the tachysterol and trans forms is suppressed in all the Formulations 1, 2, and 3 to which an antioxidant(s) were added, as compared with the control formulation.

### Example 4:

To examine an influence of the amount of an antioxidant added on the generation of the main degradation products in a preparation comprising ED-71, soft capsules filled with an MCT solution containing ED-71 with varying amounts of dl-α-tocopherol added thereto were stored under various types of conditions and evaluated for the generation behavior of the tachysterol and trans forms.

### (Formulation)

The formulation of the test soft capsule is shown in Table 5. ED-71 was mixed in the formulation in an amount of 0.001% by weight relative to MCT. dl-α-tocopherol (manufactured by Eisai Co., Ltd.) was used as an antioxidant and mixed in an amount of 0.002%, 0.005%, 0.01%, 0.02%, and 0.04% by weight relative to MCT (in Formulations 4, 5, 6, 7, and 8, respectively). A formulation free of dl-α-tocopherol was defined as Control Formulation 2.

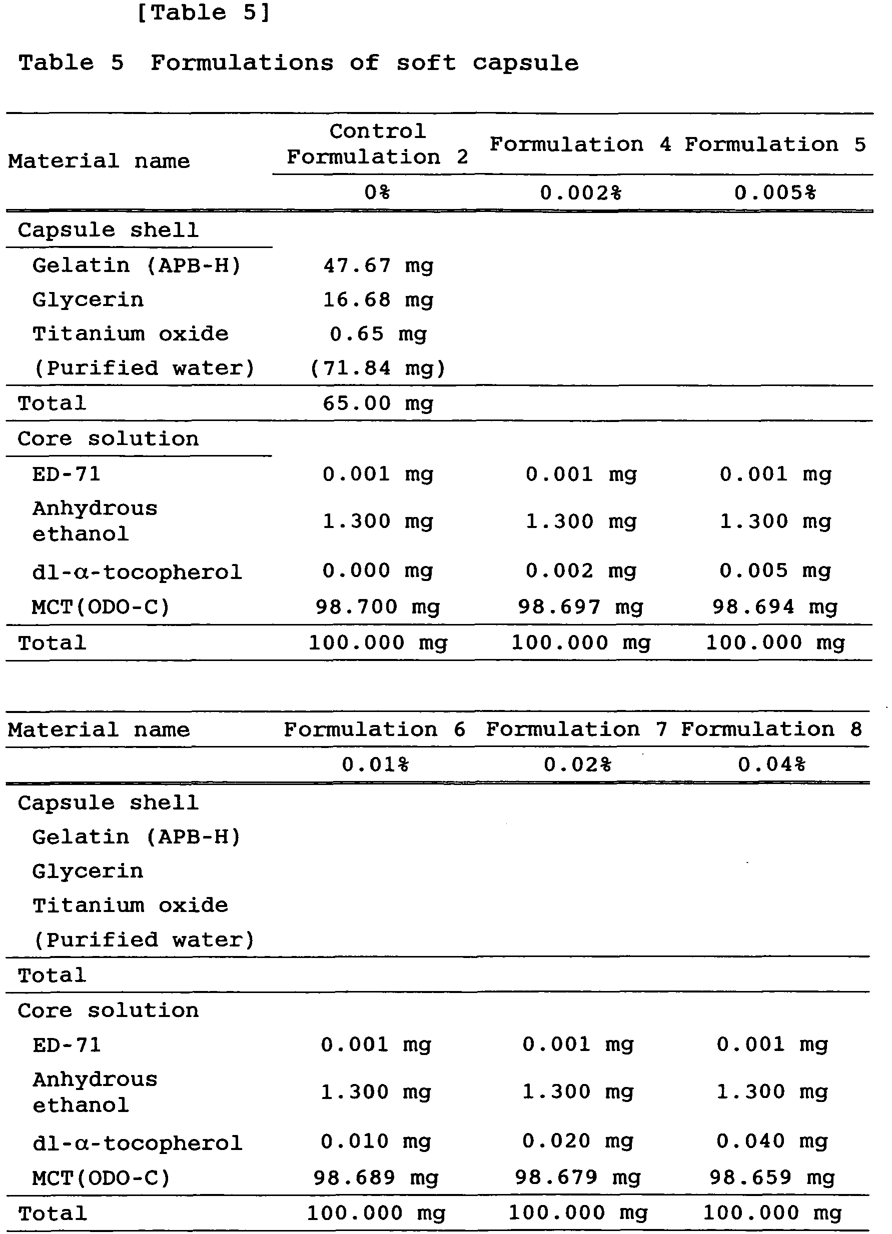

### (Production procedures for soft capsules)

The soft capsules were produced by a dripping method by means of a seamless capsule filling machine (SPHEREX, manufactured by Freund Corporation) so that the weights of the drug solution and the shell were 100 mg and 65 mg, respectively, per capsule.

### (Storage conditions and confirmation procedures for tachysterol and trans forms)

Approximately 100 capsules each of the soft capsules were put in a vial and stored at 40°C for 3 months under a shading condition, with the vial hermetically sealed. After termination of the storage period, the drug solution was extracted from each of the soft capsules, and the amounts of the tachysterol and trans forms generated were measured by the method described in Example 3.

### (Results)

The detection results of the tachysterol form and the trans form by HPLC are shown in Tables 6 and 7.
[Table 6]

**Table 6 Peak area ratio of the tachysterol form after storage of a hermetically sealed vial storing a soft capsule containing 1 µg of ED-71 at 40°C under shading**

| | | Peak area ratio of tachysterol form | | |
|---|---|---|---|---|
| Storage conditions | Storage period (month) | Control Formulation 2 | Formulation 4 | Formulation 5 |
| | | (0%) | (0.002%) | (0.005%) |
| 40°C Hermetically sealed | 0 | N.D. | N.D. | N.D. |
| | 1 | 1.22 | 0.46 | 0.27 |
| | 2 | 2.81 | 0.90 | 0.51 |
| | 3 | 4.04 | 1.34 | 0.86 |
| | | | | |

| | | Peak area ratio of tachysterol form | | |
|---|---|---|---|---|
| Storage conditions | Storage period (month) | Formulation 6 | Formulation 7 | Formulation 8 |
| | | (0.01%) | (0.02%) | (0.04%) |
| 40°C Hermetically sealed | 0 | N.D. | N.D. | N.D. |
| | 1 | 0.24 | N.D. | N.D. |
| | 2 | 0.46 | 0.28 | N.D. |
| | 3 | 0.64 | 0.42 | 0.23 |

| | | | | |
|---|---|---|---|---|
| Percentages in parentheses are the amount of dl-α-tocopherol added. n = 1, N.D. < 0.1% | | | | |

[Table 7]

**Table 7 Peak area ratio of the trans form after storage of a hermetically sealed vial storing a soft capsule containing 1 µg of ED-71 at 40°C under shading**

| | | Peak area ratio of the trans form | | |
|---|---|---|---|---|
| Storage condition | Storage period (month) | Control Formulation 2 (0%) | Formulation 4 (0.002%) | Formulation 5 (0.005%) |
| 40°C Hermetically sealed | 0 | N.D. | N.D. | N.D. |
| | 1 | 0.87 | N.D. | N.D. |
| | 2 | 2.18 | N.D. | N.D. |
| | 3 | 3.33 | 0.60 | 0.14 |

| Storage condition | Storage period | Formulation 6 | Formulation 7 | Formulation 8 |
|---|---|---|---|---|
| | (month) | (0.01%) | (0.02%) | (0.04%) |
| 40°C Hermetically sealed | 0 | N.D. | N.D. | N.D. |
| | 1 | N.D. | N.D. | N.D. |
| | 2 | N.D. | N.D. | N.D. |
| | 3 | N.D. | N.D. | N.D. |

| | | | | |
|---|---|---|---|---|
| Percentages in parentheses are the amount of dl-α-tocopherol added. n = 1, N.D. < 0.1% | | | | |

As can be seen from Tables 6 and 7, an effect of suppressing the generation of the tachysterol and trans forms was observed in Formulations 4 to 8 (even when the amount of dl-α-tocopherol added was 0.002% by weight) as compared with the control formulation. The effect of suppressing the generation of the tachysterol form was observed in such a manner that the effect was dependent on the amount of dl-α-tocopherol added. On the other hand, for the effect of suppressing the generation of the trans form, the amount of the trans form generated was 0.1% or less in all regions where the amount of dl-α-tocopherol added was 0.01% or more. These results show that dl-α-tocopherol suppresses the generation of the main degradation products of ED-71 in a concentration-dependent manner, and even when added in an amount of 0.002% by weight, has a remarkable effect of suppressing the generation of the main degradation products.

### Example 5:

To evaluate effects of various types of antioxidants on the generation of the main degradation products in a preparation comprising ED-71, MCT solutions containing ED-71 together with various types of antioxidants added were stored at 50°C for 1 month and evaluated for the generation behavior of the tachysterol and trans forms.

### (Formulation)

ED-71 was mixed in an amount of 0.0017% by weight relative to MCT in formulations. dl-α-tocopherol (manufactured by Wako Pure Chemical Industries, Ltd.), dibutylhydroxytoluene (manufactured by Wako Pure Chemical Industries, Ltd.), butylhydroxyanisole (manufactured by Wako Pure Chemical Industries, Ltd.), and propyl gallate (manufactured by Wako Pure Chemical Industries, Ltd.) were used alone as an antioxidant. The amount of each of the antioxidants added was set to 0.2% by weight relative to MCT. Formulations containing an antioxidant were defined as Formulations 9, 10, 11, and 12, while a formulation free of the antioxidant was defined as Control Formulation 3.

### (Storage conditions and confirmation procedures for the tachysterol and trans forms)

Approximately 1 g each of the drug solutions of Formulations 9 to 12 was placed into a sampling tube. The tube was hermetically sealed and stored at 50°C for 1 month under a shading condition. After the termination of the storage period, the amounts of the tachysterol and trans forms generated in each of the drug solutions were measured by the method described in Example 3.

### (Results)

The detection results of the tachysterol form and the trans form by HPLC are shown in Table 8.
[Table 8]

**Table 8 Peak area ratios of the tachysterol and trans forms after storage of an MCT solution containing ED-71 at 50°C for 1 month under a shading condition**

| | | Peak area ratio (%) | |
|---|---|---|---|
| Formulation | Antioxidant | The Tachysterol form | The Transform |
| Control | not used | 0.76 | 0.43 |
| formulation 3 | | | |
| Formulation 9 | dl-α-tocopherol | N.D. | N.D. |
| Formulation 10 | Dibutylhydroxytoluene | N.D. | N.D. |
| Formulation 11 | Butylhydroxyanisole | N.D. | N.D. |
| Formulation 12 | Propyl gallate | N.D. | N.D. |

| | | | |
|---|---|---|---|
| n = 1, N.D. < 0.1% | | | |

As can be seen from Table 8, all the antioxidants used remarkably suppressed the generation of the tachysterol and trans forms.

### Example 6

Preparations comprising ED-71 were prepared in accordance with the method described in Example 4, except that the test preparation capsules are soft capsules having the formulations described below, and evaluated for the generation behavior of the tachysterol and trans forms. All the preparations of the formulations suppressed the generation of the tachysterol and trans forms.
[Table 9]

**Table 9 Formulation of soft capsule**

| Material name | Formulation 13 | Formulation 14 |
|---|---|---|
| | (0.02%) | (0.02%) |
| Capsule shell | | |
| Gelatin (APB-H) | 54.71 mg | |
| Sorbitol | 8.34 mg | |
| Caramel (2MC) | 1.95 mg | |
| (Purified water) | (82.33 mg) | |
| Total | 65.00 mg | |
| Core solution | | |
| ED-71 | 0.0005 mg | 0.001 mg |
| Anhydrous ethanol | 1.300 mg | 1.300 mg |
| dl-α-tocopherol | 0.020 mg | 0.020 mg |
| MCT(ODO-C) | 98.6795 mg | 98.679 mg |
| Total | 100.000 mg | 100.000 mg |

### INDUSTRIAL APPLICABILITY

The method of the present invention makes it possible to provide a pharmaceutical formulation capable of suppressing the generation of a degradation product of ED-71. The trans form of ED-71 is useful as a standard in the analysis of ED-71 preparation and is also useful as a material for the synthesis of various types of vitamin D-based compounds.

## Claims

1. A method of suppressing isomerization occurring in an oily preparation comprising (5Z,7E)-(1R,2R,3R)-2-(3-hydroxypropoxy)-9,10-secocholesta-5,7,10(19)-triene-1,3,25-triol, and fat and oil, comprising adding an antioxidant to the preparation, wherein the izomerization is that (5Z,7E)-(1R,2R,3R)-2-(3-hydroxypropoxy)-9,10-secocholesta-5,7,10(19)-triene-1,3,25-triol isomerizes to 6E-(1R,2R,3R)-2-(3-hydroxypropoxy)-9,10-secocholesta-5(10),6,8(9)-triene-1,3,25-triol and/or (5E,7E)-(1R,2R,3R)-2-(3-hydroxypropoxy)-9,10-secocholesta-5,7,10(19)-triene-1,3,25-triol, and the antioxidant is added to the preparation so that the preparation comprises 0.0001 to 12% by weight of the antioxidant relative to the fat and oil,
wherein the preparation comprises 0.000001 to 0.01% by weight of (5Z,7E)-(1R,2R,3R)-2-(3-hydroxypropoxy)-9,10-secocholesta-5,7,10(19)-triene-1,3,25-triol relative to the fat and oil, and
wherein the antioxidant is selected from the group consisting of butylhydroxyanisole (BHA), catechin, dl-α-tocopherol, dibutylhydroxytoluene (BHT), and citric acid.

2. The method according to claim 1, wherein the antioxidant is dl-α-tocopherol.

## Patentansprüche

1. Verfahren zum Unterdrücken der Isomerisierung, die in einer öligen Zubereitung stattfindet, die (5Z,7E)-(1R,2R,3R)-2-(3-Hydroxypropoxy)-9,10-secocholesta-5,7,10(19)-trien-1,3,25-triol und Fett und Öl umfasst, wobei das Verfahren das Hinzufügen eines Antioxidans zu der Zubereitung umfasst, wobei die Isomerisierung darin besteht, dass (5Z,7E)-(1R,2R,3R)-2-(3-Hydroxypropoxy)-9,10-secochotesta-5,7,10(19)-trien-1,3,25-triol zu 6E-(1R,2R,3R)-2-(3-Hydroxypropoxy)-9,10-secocholesta-5(10),6,8(9)-trien-1,3,25-triol und/oder (5E,7E)-(1 R,2R,3R)-2-(3-Hydroxypropoxy)-9,10-secocholesta-5,7,10(19)-trien-1,3,25-triol isomerisiert und das Antioxidans der Zubereitung hinzugefügt wird, so dass die Zubereitung 0,0001 bis 12 Gew.-% des Antioxidans im Verhältnis zu dem Fett und Öl umfasst,
wobei die Zubereitung 0,000001 bis 0,01 Gew.-% an (5Z,7E)-(1R,2R,3R)-2-(3-Hydroxypropoxy)-9,10-secocholesta-5,7,10(19)-trien-1,3,25-triol im Verhältnis zu dem Fett und Öl umfasst, und
wobei das Antioxidans aus der Gruppe bestehend aus Butylhydroxyanisol (BHA), Catechin, DL-α-Tocopherol, Dibutylhydroxytoluol (BHT) und Zitronensäure ausgewählt ist.

2. Verfahren nach Anspruch 1, wobei das Antioxidans DL-α-Tocopherol ist.

## Revendications

1. Procédé de suppression de l'isomérisation ayant lieu dans une préparation huileuse comprenant du (5Z,7E)-(1R,2R,3R)-2-(3-hydroxypropoxy)-9,10-sécocholesta-5,7,10(19)-triène-1,3,25-triol, et de la graisse et de l'huile, comprenant l'ajout d'un antioxydant à la préparation, dans lequel l'isomérisation consiste en l'isomérisation du (5Z,7E)-(1R,2R,3R)-2-(3-hydroxypropoxy)-9,10-sécocholesta-5,7,10(19)-triène-1,3,25-triol en 6E-(1R,2R,3R)-2-(3-hydroxypropoxy)-9,10-sécocholesta-5(10),6,8(9)-triène-1,3,25-triol et/ou en (5E,7E)-(1R,2R,3R)-2-(3-hydroxypropoxy)-9,10-sécocholesta-5,7,10(19)-triène-1,3,25-triol, et l'antioxydant est ajouté à la préparation de manière à ce que la préparation comprenne de 0,0001 à 12 % en poids de l'antioxydant par rapport à la graisse et à l'huile,
dans lequel la préparation comprend de 0,000001 à 0,01 % en poids de (5Z,7E)-(1R,2R,3R)-2-(3-hydroxypropoxy)-9,10-sécocholesta-5,7,10(19)-triène-1,3,25-triol par rapport à la graisse et à l'huile, et
dans lequel l'antioxydant est choisi dans le groupe constitué du butylhydroxyanisole (BHA), de la catéchine, du dl-α-tocophérol, du dibutylhydroxytoluène (BHT) et de l'acide citrique.

2. Procédé selon la revendication 1, dans lequel l'antioxydant est le dl-α-tocophérol.
